# EUROPEAN PATENT APPLICATION

(11) **EP 4 273 875 A1**
(43) Date of publication of application: **08.11.2023**
(21) Application number: 21914568.7
(22) Date of filing: 29.12.2021
(51) Int. Cl.: G16H 40/40, G16H 50/70, G06F 16/903

(54) **RADIO FREQUENCY ABLATION DATA PROCESSING METHOD AND APPARATUS, SERVER, AND COMPUTER-READABLE STORAGE MEDIUM**

(30) Priority: 31.12.2020 CN 202011639895
(71) Applicant: Hangzhou Broncus Medical Co., Ltd., Hangzhou, Zhejiang 310051 (CN)
(72) Inventor: CUI, Changjie, Hangzhou, Zhejiang 310051 (CN); XU, Hong, Hangzhou, Zhejiang 310051 (CN)
(74) Representative: MERH-IP Matias Erny Reichl Hoffmann Patentanwälte PartG mbB
(86) International application number: PCT/CN2021/142747
(87) International publication number: WO 2022/143836

(57) **Abstract**

A radio frequency ablation data processing method and apparatus, a server, and a computer-readable storage medium are provided. The method includes: acquiring radio frequency ablation data of each of the ablation tasks performed in a preset period of time, wherein the radio frequency ablation data includes configured parameter data for each device in a radio frequency ablation system when each of the ablation tasks is performed, and characteristic data of an ablation object associated with each of the ablation tasks; and analyzing the radio frequency ablation data, to obtain and output respective ablation parameter configuration schemes corresponding to various types of ablation objects. By using the radio frequency ablation data processing method and apparatus, the server and the computer-readable storage medium, analysis of radio frequency ablation data based on big data is realized, and reference for ablation parameter configuration is provided for various types of ablation objects.

## Description

### TECHNICAL FIELD

Embodiments of the present invention relates to the technical field of data processing, and particularly to a radio frequency ablation data processing method and apparatus, a server, and a computer-readable storage medium.

### DESCRIPTION OF THE PRIOR ART

Radio frequency ablation (RFA) is a common technology for minimally invasive ablation of tumors. The principle of radio frequency ablation is to use an alternating highfrequency current with a frequency of less than 30 MHz to cause high-speed oscillations and frictions of ions in tumor tissue, so the radio frequency energy is converted into heat energy, causing coagulative necrosis of tumor cells.

When each ablation task is performed, each device in the radio frequency ablation system will generate lots of radio frequency ablation data. In the prior art, the radio frequency ablation data is only recorded in the log. How to use the radio frequency ablation data to provide data reference and technical support for future ablation tasks is an important problem to be solved urgently in the industry at present.

### SUMMARY OF THE DISCLOSURE

Embodiments of the present invention provide a radio frequency ablation data processing method and apparatus, a server, and a non-transitory computer-readable storage medium. By the present invention, analysis of radio frequency ablation data based on big data can be realized, and reference for ablation parameter configuration can be provided for various types of ablation objects, so as to improve the utilization of the radio frequency ablation data.

In an aspect, an embodiment of the present invention provides a radio frequency ablation data processing method, for use in a computer device. The method includes:
acquiring radio frequency ablation data of each of the ablation tasks performed in a preset period of time, wherein the radio frequency ablation data includes configured parameter data for each device in a radio frequency ablation system when each of the ablation tasks is performed, and characteristic data of an ablation object associated with each of the ablation tasks; and
analyzing the radio frequency ablation data, to obtain and output respective ablation parameter configuration schemes corresponding to various types of ablation objects.

In an aspect, an embodiment of the present invention further provides a radio frequency ablation data processing apparatus. The apparatus includes:
an acquisition module, configured to acquire radio frequency ablation data of each of the ablation tasks performed in a preset period of time, wherein the radio frequency ablation data includes configured parameter data for each device in a radio frequency ablation system when each of the ablation tasks is performed, and characteristic data of an ablation object associated with each of the ablation tasks;
an analysis module, configured to analyze the radio frequency ablation data, to obtain respective ablation parameter configuration schemes corresponding to various types of ablation objects; and
an output module, configured to output the ablation parameter configuration scheme.

In an aspect, an embodiment of the present invention further provides a server, which includes a storage and a processor, wherein
the storage stores an executable program code; and
the processor is coupled to the storage, and configured to call the executable program code stored in the storage, and implement the radio frequency ablation data processing method provided in the above embodiment.

In an aspect, an embodiment of the present invention further provides a non-transitory computer-readable storage medium storing a computer program. When the computer program is executed by the processor, the radio frequency ablation data processing method provided in the above embodiment is implemented.

In various embodiments provided in the present invention, radio frequency ablation data of each of the ablation tasks performed in a preset period of time is acquired, and the acquired radio frequency ablation data is analyzed, to obtain respective ablation parameter configuration schemes corresponding to various types of ablation objects. As a result, automatic analysis of radio frequency ablation data based on big data and recommending of an ablation parameter configuration scheme based on the automatic analysis are realized, so as to improve the utilization of the radio frequency ablation data. Moreover, the analysis result is obtained based on massive data, and thus has a high reference value.

### BRIEF DESCRIPTION OF DRAWINGS

In order to describe the technical solutions according to the embodiments of the present invention or in the prior art more clearly, the drawings needed to be used in the embodiments or in the prior art will be described briefly below. Apparently, the drawings in the following description show some embodiments of the present invention. Other drawings can be obtained by persons of ordinary skill in the art based on these drawings without creative efforts.
FIG. 1 shows an application environment of a radio frequency ablation data processing method provided in an embodiment of the present invention;
FIG. 2 shows a flow chart of implementing a radio frequency ablation data processing method provided in an embodiment of the present invention;
FIG. 3 shows a flow chart of implementing a radio frequency ablation data processing method provided in another embodiment of the present invention;
FIG. 4 is a structural schematic view of a radio frequency ablation data processing apparatus provided in an embodiment of the present invention;
FIG. 5 is a structural schematic view of a radio frequency ablation data processing apparatus provided in another embodiment of the present invention; and
FIG. 6 is a schematic diagram showing a hardware structure in a server provided in an embodiment of the present invention.

### DESCRIPTION OF EMBODIMENTS

In order to make the objects, technical solutions and advantages of the embodiments of the present invention clearer, the technical solutions according to the embodiments of the present invention will be clearly and completely described below with reference to drawings in the embodiments of the present invention. Apparently, the embodiments described are merely some embodiments, but not all of the embodiments of the present invention. All other embodiments obtained by ordinary persons skilled in the art based on the embodiments of the present invention without creative efforts shall fall within the protection scope of the present invention.

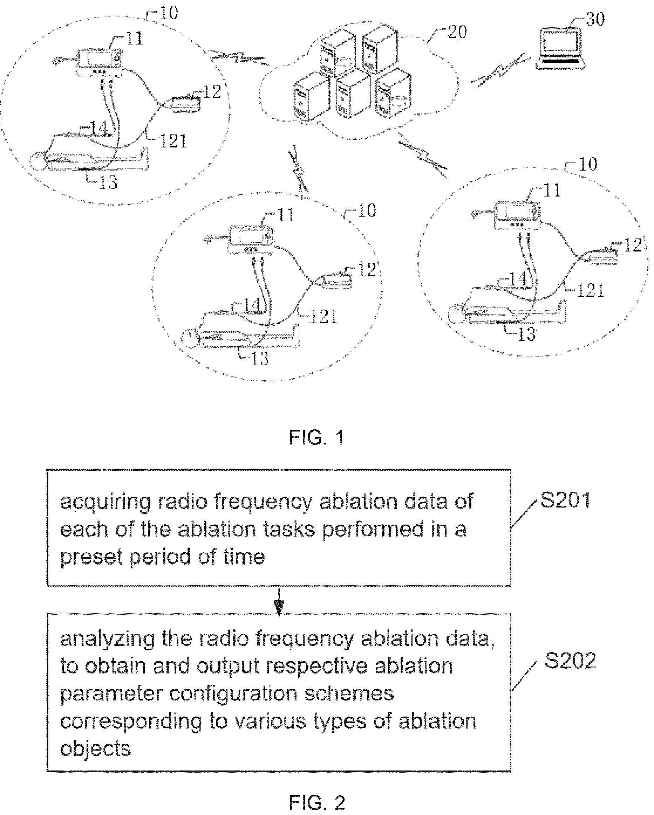

FIG. 1 shows an application scenario of a radio frequency ablation data processing method provided in an embodiment of the present invention. The radio frequency ablation data processing method can be implemented by a server 20 shown in FIG. 1. The server 20 may be a single cloud server, or a distributed server cluster consisting of multiple servers in the cloud. The distributed server cluster includes at least one access server group for data access, at least one distribution server group for data distribution, at least one cloud computing server group for data processing and at least one database server group for data storage (for example, database in various embodiments of the present invention). The server groups can be configured in the same place, or distributed and configured at various positions.

The access server group serves as an external interface of the distributed server cluster, and establishes, through a first gateway, data connection with the distribution server group for data exchange, to achieve data acquisition and data distribution in the radio frequency ablation data processing method provided in various embodiments of the present invention.

The distribution server group establishes, through a second gateway, data connection with the cloud computing server group for data exchange, to achieve distribution of radio frequency ablation data and control of data processing in the radio frequency ablation data processing method provided in various embodiments of the present invention. The distribution server group determines, according to a real-time processing capacity of each cloud computing server, a target server for processing data sent from the access server group.

The cloud computing server group establishes, through a third gateway, data connection with the database server group, to achieve the analysis and processing of radio frequency ablation data in the radio frequency ablation data processing method provided in various embodiments of the present invention, and achieve the storage query and modification of data in a database in various embodiments of the present invention.

The server 20 establishes, through a wireless network or by wired connection, data connection with at least one device in one or more radio frequency ablation systems 10 and an additional related apparatus 30; acquires, through the data connection, radio frequency ablation data of an ablation task performed by each radio frequency ablation system 10; and processes the radio frequency ablation data by a radio frequency ablation data processing method provided in the following embodiments. For convenience of understanding, FIG. 1 merely shows three radio frequency ablation systems 10. The present invention is not limited thereto in practical use. Multiple radio frequency ablation systems 10 can be respectively configured at different locations, for example, in different departments of multiple entities in different regions.

It can be understood that when the server 20 only establishes data connection with some devices in the radio frequency ablation system 10, these devices can serve as an interface, to forward radio frequency ablation data of other devices in the radio frequency ablation system 10 to the server 20.

The additional related apparatus 30 may be, for example, a medical imaging apparatus, an intelligent physical examination apparatus, a personal computer terminal of a doctor, a server of a medical data public platform, and other radio frequency ablation data acquisition and storage apparatus.

As shown in FIG. 1, the radio frequency ablation system 10 includes a radio frequency ablation control device 11, an injection pump 12, a neutral electrode 13 and a radio frequency ablation catheter 14.

Before an ablation task is implemented, the radio frequency ablation catheter 14 configured to generate and output radio frequency energy and an extension tube 121 of the injection pump 12 are inserted into an ablation object (such as a patient with emphysema), reaching an ablation site. Then, the neutral electrode 13 is brought into contact with the skin surface of the ablation object, and a radio frequency current flows through the radio frequency ablation catheter 14, such that the tissue of the ablation object and the neutral electrode 13 form a circuit loop.

When the ablation task is triggered, the radio frequency ablation catheter 14 is controlled by the radio frequency ablation control device 11 to output radio frequency energy to the ablation site by single-electrode discharging, so as to implement an ablation operation on the ablation site. Meanwhile, the injection pump 12 performs an injection operation on the ablation object through the extension tube 121, and physiological saline is injected into the ablation site, to adjust the impedance and temperature of the ablation site.

FIG. 2 shows a flow chart of implementing a radio frequency ablation data processing method provided in an embodiment of the present invention. The method can be implemented by a computer device, such as the server 20 shown in FIG. 1. As shown in FIG. 2, the method specifically includes the following steps:

Step S201: acquiring radio frequency ablation data of each of the ablation tasks performed in a preset period of time.

Particularly, the server periodically acquires radio frequency ablation data of each of the ablation tasks performed in a preset period of time. The radio frequency ablation data includes configured parameter data for each device in at least one radio frequency ablation system when each of the ablation tasks is performed, and characteristic data of an ablation object associated with each of the ablation tasks. The preset period of time may be, for example, one month, half a year, one year, or longer, or a time interval from the time when each radio frequency ablation system is put into use to the time when the server acquires the radio frequency ablation data, which can be specifically set according to the user's custom operation.

The parameter data may include, but is not limited to, for example, a radio frequency power, an ablation time, and an alarm value; a liquid injection volume, an injection time, and an alarm value of the injection pump, and so on, called when the radio frequency ablation control device controls the radio frequency ablation catheter to perform an ablation operation. The radio frequency ablation control device and the injection pump perform the ablation task according to their respective parameter data.

The characteristic data of the ablation object may include, but is not limited to, for example, the name, age, gender, disorder, and others of the ablation object.

The server can acquire the parameter data by periodically sending a data acquisition request to a device in the radio frequency ablation system. Alternatively, a device in the radio frequency ablation system can also report the configured parameter data when each of the ablation tasks is implemented to the server in real time or periodically. Alternatively, a device in the radio frequency ablation system can also report the configured parameter data when each of the ablation tasks is implemented to a data acquisition server dedicated for the acquisition of radio frequency ablation data in real time or periodically, and the server periodically acquires the parameter data from the data acquisition server.

It can be understood that the characteristic data of the ablation object associated with each of the ablation tasks can be stored in the radio frequency ablation system, or the data acquisition server. When stored in the data acquisition server, the server can query the characteristic data of the ablation object associated with the ablation task from the data acquisition server according to identification information of the ablation task.

The data acquisition server can obtain the characteristic data of the ablation object associated with each ablation task according to an input operation of a user, or obtain a description file (for example, an electronic prescription) of each ablation task from other terminals (for example, computer terminals in various medical clinics) and then extract the characteristic data of the ablation object associated with each ablation task from the description file.

Step S202: analyzing the radio frequency ablation data, to obtain and output respective ablation parameter configuration schemes corresponding to various types of ablation objects.

Particularly, the ablation objects are classified according to the characteristic data of each ablation object. Then, the radio frequency ablation data is classified according to the type of the ablation object associated with the corresponding ablation task, to obtain and output respective ablation parameter configuration schemes corresponding to various types of ablation objects.

Optionally, all pieces of parameter data of all the devices in the radio frequency ablation system called for implementing the same ablation task can be used as an ablation parameter configuration scheme, or all pieces of parameter data of one device in the radio frequency ablation system called for implementing one ablation task can be used as an ablation parameter configuration scheme.

In the embodiment of the present invention, radio frequency ablation data of each of the ablation tasks performed in a preset period of time is acquired, and the acquired radio frequency ablation data is analyzed, to obtain respective ablation parameter configuration schemes corresponding to various types of ablation objects. As a result, automatic analysis of radio frequency ablation data based on big data and recommending of an ablation parameter configuration scheme based on the automatic analysis are realized, so as to improve the utilization of the radio frequency ablation data. Moreover, the analysis result is obtained based on massive data, and thus has a high reference value.

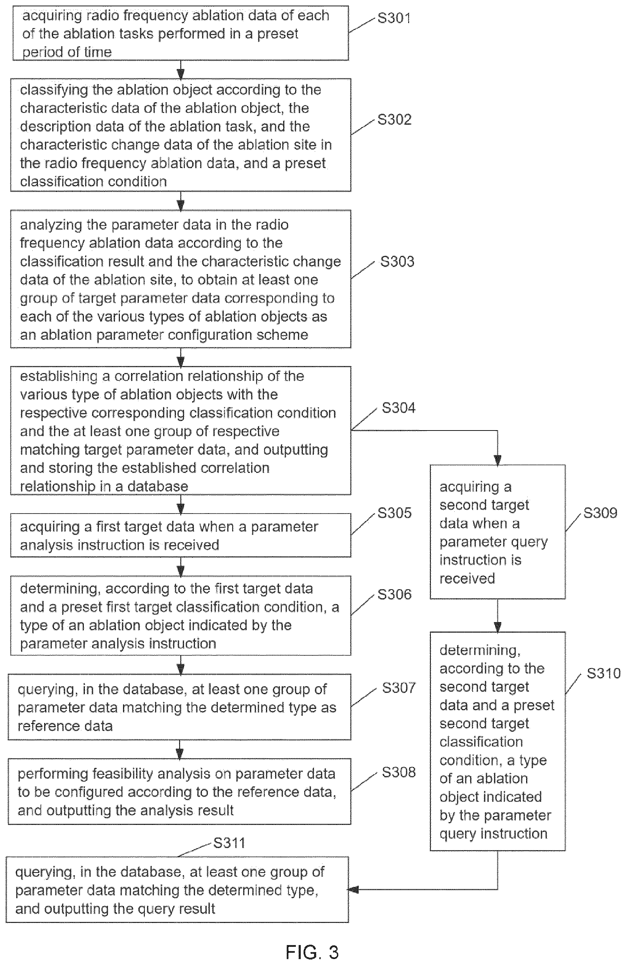

FIG. 3 shows a flow chart of implementing a radio frequency ablation data processing method provided in an embodiment of the present invention. The method can be implemented by a computer device, such as the server 20 shown in FIG. 1. As shown in FIG. 3, the method specifically includes the following steps:

Step S301: acquiring radio frequency ablation data of each of the ablation tasks performed in a preset period of time.

Particularly, the server periodically acquires radio frequency ablation data of each of the ablation tasks performed in a preset period of time. The radio frequency ablation data includes configured parameter data for each device in a radio frequency ablation system when each of the ablation tasks is performed, and characteristic data of an ablation object associated with each of the ablation tasks. The preset period of time may be, for example, one month, half a year, one year, or longer, or a time interval from the time when the radio frequency ablation system is put into use to the time when the server acquires the radio frequency ablation data, which can be specifically set according to the user's custom operation.

The radio frequency ablation data includes the configured parameter data for each device in the radio frequency ablation system when each of the ablation tasks is performed, the characteristic data of the ablation object associated with each of the ablation tasks, description data of each of the ablation tasks, characteristic data of an ablation site of each of the ablation objects, and characteristic change data of each of the ablation sites after the respectively associated ablation task is implemented.

The parameter data may include, but is not limited to, for example, a radio frequency power, an ablation time, and an alarm value of the radio frequency ablation control device; a liquid injection volume, an injection time, a flow rate, and an alarm value of the injection pump, and so on. The radio frequency ablation control device and the injection pump perform the ablation task according to their respective parameter data.

Optionally, the characteristic data of the ablation object may include, but is not limited to, at least one of the gender, the disorder before the ablation task is implemented and the survival time after the ablation task is implemented, and the age of the ablation object. The disorder includes other disorders than the disorder corresponding to the ablation task, such as hypertension, high fever, asthma and so on.

Optionally, the description data of the ablation task includes description data of implementation time of the ablation task and the ablation stage corresponding to the ablation task, for example, the first ablation task, or an nth ablation stage.

Optionally, the characteristic change data of the ablation site include the change data of at least one of the position, size, shape and area of the ablation site before and after each of the associated ablation tasks is performed.

The server can acquire the parameter data by periodically sending a data acquisition request to a device in the radio frequency ablation system. Alternatively, a device in the radio frequency ablation system can also report the configured parameter data when each of the ablation tasks is implemented to the server in real time or periodically. Alternatively, the device in the radio frequency ablation system can also report the configured parameter data when each of the ablation tasks is implemented to a data acquisition server dedicated for the acquisition of radio frequency ablation data in real time or periodically, and the server periodically acquires the parameter data from the data acquisition server.

It can be understood that in the radio frequency ablation data, other data than the parameter data can be stored in the radio frequency ablation system, or in the dedicated data acquisition server. When stored in the data acquisition server, the server can query the other radio frequency ablation data associated with the ablation task from the data acquisition server according to identification information of the ablation task and identification information of the ablation object. The identification information of the ablation task is used to uniquely identify the identity of the ablation task, such as a global unique number of the ablation task. The identification information of the ablation object is used to uniquely identify the identity of the ablation object, such as the name of the ablation object. These pieces of identification information can be allocated by the radio frequency ablation system or other related apparatuses in implementing the corresponding task, and reported to the server. Alternatively, these pieces of identification information can also be allocated by the server and sent to the radio frequency ablation system or other related apparatuses.

The data acquisition server can acquire the radio frequency ablation data according to an input operation of a user, or acquire the radio frequency ablation data from other terminals (for example, computer terminals in various medical clinics), for example, by obtaining a description file (for example, an electronic prescription) of each ablation task from other terminals and then extracting the characteristic data of the ablation object associated with each ablation task from the description file.

Optionally, in another embodiment of the present invention, the method further includes:
receiving the identification information of the ablation object, imaging time, and imaging data of the ablation object sent from a medical imaging apparatus; performing image recognition on the imaging data, and generating characteristic data of the ablation site of the ablation object according to the recognition result; and correlating the identification information of the ablation object and the imaging time with the characteristic data of the ablation site, and storing the correlation relationship in an ablation site information database.

The medical imaging apparatus may include, but is not limited to, an X-ray machine, and a Computed Tomography (CT) machine. Particularly, the medical imaging apparatus can report, after each imaging task is implemented or periodically, the imaging data obtained when the imaging task is performed to the server.

The characteristic data of the ablation site may include, but is not limited to, at least one of the position, size, shape and area of the ablation site. Optionally, the ablation site information database can be configured in a cloud data acquisition server.

Then the characteristic change data for the ablation site of each ablation object can also be obtained by querying the ablation site information database, to obtain the characteristic data of the ablation site of each of the ablation objects and the corresponding imaging time; and the characteristic change data for the ablation site of each ablation object are obtained according to the imaging time.

Particularly, the server can obtain the characteristic data of the ablation sites of all the ablation objects and the corresponding imaging time at a time by querying the ablation site information database, or query the characteristic data of one or more corresponding ablation objects and the imaging time according to the identification information of one or more ablation objects.

In this way, other related apparatuses such as a medical imaging apparatus are incorporated into a network for automatic analysis of the radio frequency ablation data, to realize the automatic collection of related data, thus saving the labor cost, shortening the data acquisition time, and further improving the efficiency of data analysis.

Step S302: classifying the ablation object according to the characteristic data of the ablation object, the description data of the ablation task, and characteristic change data of the ablation site in the radio frequency ablation data, and a preset classification condition.

Specifically, the preset classification condition is criterion for classification. According to specific contents of the radio frequency operation data, as shown in Table 1 below, the criterion for classification may include, but is not limited to, for example, the age, gender, disorder before an ablation task is implemented, ablation stage, characteristic range of the ablation site, and survival time. In practical use, one or more classification conditions can be preset according to the user's custom operation.

In this way, the ablation object is classified according to the preset classification condition, to distinguish various types of ablation objects; and then the radio frequency ablation data is analyzed according to the classification result, such that the ablation parameter configuration scheme obtained after analysis has high pertinence and reference value.

**Table 1**

| Type of ablation object | Characteristic range of ablation object | | Surviva l time | Disorder | Characteristic range of ablation site | | | Ablation stage |
|---|---|---|---|---|---|---|---|---|
| | Age | Gender | | | Ar ea | Shape | Position (x, y, z) | |
| Type 1 | 0-10 years | Female | 1-3 years | No | 0.5 cm | Irregular | 3, 4, 5, | 1 |
| Type 2 | 10-20 years | Male | <1 year | Gastritis | 0.1 cm | Elliptical | 10, 11, 13 | 3 |
| ...... | | | | | | | | |

As shown in Table 1 above, multiple types of ablation objects are preset. Each type (for example, Type1, and Type 2) can correspond to one or more classification conditions (such as age, and gender, etc.). The characteristic data of the ablation object, the description data of the ablation task, and the characteristic change data of the ablation site in the radio frequency ablation data of an ablation task are respectively compared with the classification condition corresponding to various preset types, to determine a preset type corresponding to the classification condition that is most satisfied by the ablation task. The preset type is taken as the type of the ablation object. The configured parameter data when the ablation task is implemented is correlated with the preset type.

In Table 1 above, (x, y, z) are the position coordinates of the ablation site. Optionally, a two-dimensional or three-dimensional coordinate system is established by taking a certain vertex of the whole tissue and organ where the ablation site is located as the origin. The coordinates of the center of mass of the ablation site in the two-dimensional or three-dimensional coordinate system are taken as the position of the ablation site. By using the coordinates, the position of the ablation site can be located more accurately, thereby further improving the accuracy of the analysis result.

It can be understood that Table 1 above merely shows an example. In practical use, a related data table can have more or less content, and can also be presented in other forms.

Step S303: analyzing the parameter data in the radio frequency ablation data according to the classification result and the characteristic change data of the ablation site, to obtain at least one group of target parameter data corresponding to each of the various types of ablation objects as an ablation parameter configuration scheme.

Particularly, the parameter data is classified according to the classification result of the ablation object, to obtain at least one group of candidate parameter data corresponding to each of the various types of ablation objects. Then, parameter data in the candidate parameter data where the characteristic change data of the ablation site and/or the survive time does not reach a preset change standard is excluded, to obtain at least one group of target parameter data as an ablation parameter configuration scheme.

The change standard is used to evaluate the ablation effect, and it may be an absolute value, or a relative value. The change standard can correspond to at least one of the characteristic change data of the ablation site, for example, a preset value to which the size of the ablation site to be reduced (for example, reduced to 0.01 cm), or a value by which the area of the ablation site to be reduced (for example, reduced by 0.1 cm). The type and parameter value of the change standard can be specifically set according to the user's custom operation.

Optionally, in another embodiment of the present invention, the method further includes: setting an average of the characteristic change data of all the ablation sites as the preset change standard.

Namely, when the characteristic change of an ablation site reaches an average change level of all the ablation sites, for example, the area change reaches an average area change level and the size change reaches an average size change level, the corresponding parameter data can be reserved. In this way, when an ablation task is implemented by a radio frequency ablation system, all the parameter data of various devices configured in the system are used as a group of parameter data, and each group of parameter data is screened by using the ablation effect, to further improve the reference value of the analysis results.

Optionally, in another embodiment of the present invention, the method further includes analyzing normal distribution characteristics of the characteristic change data of the ablation site, and setting a normal distribution characteristic value obtained after analysis as the preset change standard.

Particularly, the normal distribution is of a bell-shaped curve, having a high probability density in the middle, low probability densities at two sides, and a shape determined by the parameters µ and σ. A continuous random variable conforming a normal distribution with a mean µ and a standard deviation σ is expressed as X~N(µ, σ^2), wherein µ refers to the central position of the curve, and generally, the maximum probability density appears near the mean. By analyzing the normal distribution characteristics of the characteristic change data of the ablation site, the characteristic value with the maximum probability allowing the ablation site to have a normal distribution is obtained, and then it is set as the preset change standard, to further improve the reference value of the analysis results.

Step S304: establishing a correlation relationship of each of the various type of ablation objects with the respective corresponding classification condition and the at least one group of respective matching target parameter data, and outputting and storing the established correlation relationship in a database.

In an example of practical application, the analysis result are shown in Table 2.

**Table 2**

| Type of ablation object | Classification condition | Matching parameter data |
|---|---|---|
| Type 1 | Aged 0-10 years; the ablation site is reduced by 0.5 cm in the 1st ablation stage; | |
| | | First group: |
| | | Power of radio frequency ablation |
| | | catheter: A |
| | | Injection volume of injection pump: B |
| | ...... | |
| Type 2 | Aged 11-15 years; the ablation site is reduced by 0.1 cm in the 2nd ablation stage; | First group: |
| | | Power of radio frequency ablation |
| | | catheter: C |
| | | Injection volume of injection pump: D |
| | | Second group: |
| | | Power of radio frequency ablation |
| | ...... | catheter: E |
| | | Injection volume of injection pump: F |
| | | ...... |
| ...... | | |

It can be understood that Table 2 above merely shows an example. In practical use, a related data table can have more or less content, and can also be presented in other forms. When the correlation relationship involved in the present invention is stored in the database, besides the correlation relationship, the corresponding various types of ablation objects, classification condition, and target parameter data can also be stored in the database. Alternatively, only the storage link of the above data is saved in the database, and the source data of the above data is stored in other database servers, to reduce the volume of the database, and improve the query speed.

Optionally, in another embodiment of the present invention, the method further includes:
monitoring physiological changes of each of the ablation objects by an intelligent physical examination apparatus, to obtain physiological change data of each of the ablation objects after each of the associated ablation tasks is implemented; and
screening the target parameter data in the database periodically according to the physiological change data.

Optionally, the physiological change data may include, but is not limited to, at least one of body temperature, blood pressure, blood glucose, heart rate, blood lipid, vital capacity, and blood oxygen saturation. The intelligent physical examination apparatus may be a household physical examination apparatus, or a physical examination apparatus provided in various medical facilities, specifically for example, a smart watch, a smart bracelet, and other smart wearable devices having blood pressure, heart rate, and body temperature measuring functions, an intelligent blood glucose detector with data transmission function, an intelligent blood pressure meter, an intelligent thermometer and so on.

Further, the step of screening the target parameter data in the database periodically according to the physiological change data specifically includes
obtaining a physiologically abnormal change rate periodically according to the physiological change data; and
adjusting the storage state of the target parameter data in the database according to the physiologically abnormal change rate and a preset rate.

The physiologically abnormal change rate is a proportion of the number of the same type of ablation objects whose physiological data changes beyond a preset range in the total number of the type of ablation objects after the associated ablation task is implemented. For example, in ablation objects of Type 1, the physiologically abnormal change rate is a proportion of the number of ablation objects whose blood pressure value is continuously higher than a preset value for over a preset period of time in the total number of ablation objects of Type 1 after the associated ablation task is implemented.

The step of adjusting the storage state of the target parameter data in the database specifically includes: marking the parameter data in the target parameter data with a physiologically abnormal change rate that is greater than the preset rate as hidden, locked, or frozen in the database, such that the parameter data cannot be queried by an external device; and marking the parameter data in the target parameter data with a physiologically abnormal change rate that is not greater than the preset rate as normal, such that the parameter data can be queried by an external device.

In this way, the physiological changes of each of the ablation objects are monitored by the intelligent physical examination apparatus, and the storage state of the ablation parameter configuration scheme stored in the database is dynamically adjusted, such that the finally obtained ablation parameter configuration scheme is more pertinent and accurate with increasing volume of data.

Step S305: acquiring a first target data when a parameter analysis instruction is received.

Step S306: determining, according to the first target data and a preset first target classification condition, a type of an ablation object indicated by the parameter analysis instruction.

Step S307: querying, in the database, at least one group of parameter data matching the determined type as reference data.

Step S308: performing feasibility analysis on parameter data to be configured according to the reference data, and outputting the analysis result.

Particularly, the parameter analysis instruction can be sent to the server by other terminals according to the user's operation. When the parameter analysis instruction is sent by other terminals to the server, the first target data inputted by the user or a storage link of the first target data can also be sent to the server.

The first target data includes characteristic data of the ablation object, characteristic data of an ablation site, description data of an ablation task to be implemented, and parameter data to be configured indicated by the parameter analysis instruction.

The characteristic data of the ablation object, the characteristic data of the ablation site, the description data of the ablation task to be implemented, and the parameter data to be configured indicated by the parameter analysis instruction, the first target classification condition, and the step of determining a type of an ablation object indicated by the parameter analysis instruction according to the first target data and a first target classification condition can be specifically made reference to related description in Steps S301 and S302, and will not be repeated here again.

Particularly, the step of performing feasibility analysis on parameter data to be configured according to the reference data includes comparing the reference data with the parameter data to be configured, and determining whether the difference therebetween is less than a preset difference range; if the difference is less than the preset difference range, determining the corresponding parameter data to be configured to be feasible; and if the difference is not less than the preset difference range, determining the corresponding parameter data to be configured to be unfeasible.

The analysis result includes description information indicating whether the parameter data to be configured is feasible. Further, the analysis result may further include reference data corresponding to the unfeasible parameter data to be configured.

For example, assuming that the parameter data to be configured includes injection volume and injection duration of the injection pump, power of the radio frequency ablation catheter, and ablation duration, and according to the queried reference data, the injection volume and injection duration of the injection pump in the parameter data to be configured are feasible, and the power of the radio frequency ablation catheter and the ablation duration are unfeasible, then the analysis result includes, in addition to the description information indicating whether the parameter data to be configured is feasible, reference data for the power of the radio frequency ablation catheter and the ablation duration.

In this way, by using the reference data in the database, the configured parameter data for the ablation task to be implemented is analyzed, so that the accuracy of parameter configuration for the radio frequency ablation system can be improved, thereby achieving a better ablation effect.

Step S309: acquiring a second target data when a parameter query instruction is received.

Step S310: determining, according to the second target data and a preset second target classification condition, a type of an ablation object indicated by the parameter query instruction.

Step S311: querying, in the database, at least one group of parameter data matching the determined type, and outputting the query result.

Particularly, the second target data includes characteristic data of the ablation object, characteristic data of an ablation site, and description data of an ablation task to be implemented indicated by the parameter query instruction.

The characteristic data of the ablation object, the characteristic data of the ablation site, and the description data of the ablation task to be implemented indicated by the parameter query instruction, the second target classification condition, and the step of determining a type of an ablation object indicated by the parameter query instruction according to the second target data and a second target classification condition can be specifically made reference to related description in Steps S301 and S302, and will not be repeated here again.

Optionally, the first target classification condition and the second target classification condition respectively correspond to the first target data and the second target data. In the process of parameter analysis and parameter query, the first and second target classification condition and the scope of types of various parameters in the target data used may be less than or equal to the classification condition and the scope of types of the radio frequency ablation data used in Step S301 and Step S302. For example, in Step S301 and Step S302, the age, gender, and area of the ablation site are used as the classification conditions, and the first target classification condition can be merely age.

As a result, by using the database, a query service for an ablation parameter configuration scheme based on the target classification condition and the target data can be provided to a user. Therefore, the radio frequency ablation data is fully utilized.

In the embodiment of the present invention, radio frequency ablation data of each of the ablation tasks performed in a preset period of time is acquired, and the acquired radio frequency ablation data is analyzed, to obtain respective ablation parameter configuration schemes corresponding to various types of ablation objects. As a result, automatic analysis of radio frequency ablation data based on big data and query and analysis of an ablation parameter configuration scheme based on the automatic analysis are realized, so as to improve the utilization of the radio frequency ablation data. Moreover, the analysis result is obtained based on massive data, and thus has a high reference value.

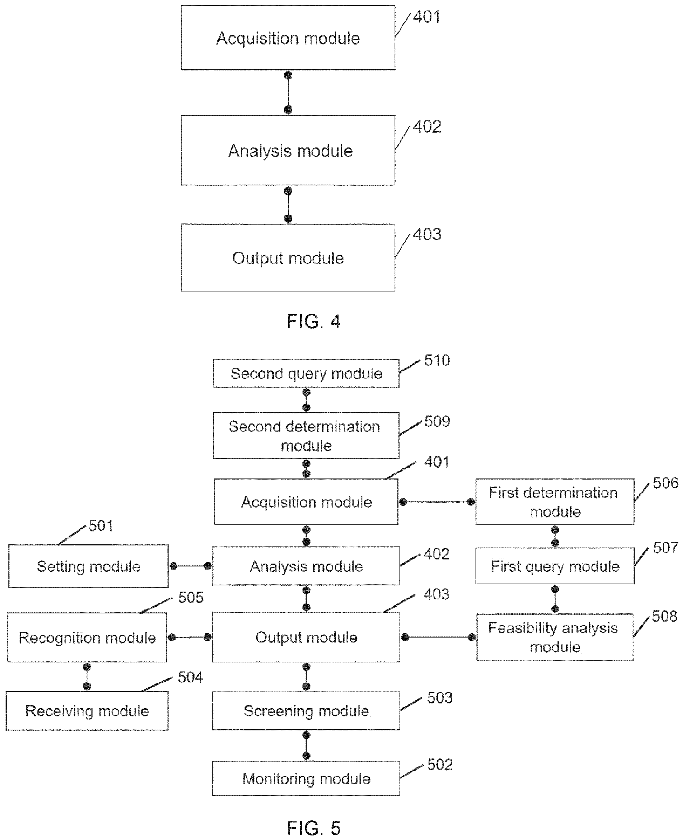

FIG. 4 is a structural schematic view of a radio frequency ablation data processing apparatus provided in an embodiment of the present invention. For convenience of description, only the parts relevant to the embodiments of the present invention are shown. The apparatus can be a server, or a software module configured in a server. As shown in FIG. 4, the apparatus includes an acquisition module 401, an analysis module 402 and an output module 403.

The acquisition module 401 is configured to acquire radio frequency ablation data of each of the ablation tasks performed in a preset period of time, wherein the radio frequency ablation data includes configured parameter data for each device in a radio frequency ablation system when each of the ablation tasks is performed, and characteristic data of an ablation object associated with each of the ablation tasks.

The analysis module 402 is configured to analyze the radio frequency ablation data, to obtain respective ablation parameter configuration schemes corresponding to various types of ablation objects.

The output module 403 is configured to output the ablation parameter configuration scheme.

The specific process for the above modules to implement their respective functions can be made reference to the relevant description in the embodiments shown in FIG. 2 and FIG. 3, and will not be repeated here again.

In the embodiment of the present invention, radio frequency ablation data of each of the ablation tasks performed in a preset period of time is acquired, and the acquired radio frequency ablation data is analyzed, to obtain respective ablation parameter configuration schemes corresponding to various types of ablation objects. As a result, automatic analysis of radio frequency ablation data based on big data and query and analysis of an ablation parameter configuration scheme based on the automatic analysis are realized, so as to improve the utilization of the radio frequency ablation data. Moreover, the analysis result is obtained based on massive data, and thus has a high reference value.

FIG. 5 is a structural schematic view of a radio frequency ablation data processing apparatus provided in another embodiment of the present invention. For ease of description, only the parts relevant to the embodiments of the present invention are shown. The apparatus can be a server, or a software module configured in a server.

Different from the embodiment shown in FIG. 4, as shown in FIG. 5, the radio frequency ablation data further includes description data of each of the ablation tasks and characteristic change data of an ablation site of each of the ablation objects.

The analysis module 402 is further configured to classify the ablation object according to the characteristic data of the ablation object, the description data of the ablation task, the characteristic change data of the ablation site, and a preset classification condition; is further configured to analyze the parameter data according to the classification result and the characteristic change data of the ablation site, to obtain at least one group of target parameter data corresponding to each of the various types of ablation objects as the ablation parameter configuration scheme; and is further configured to establish a correlation relationship of the various type of ablation objects with the respective corresponding classification condition and the at least one group of respective matching target parameter data.

The output module 403 is further configured to output and store the established correlation relationship in a database.

Further, the analysis module 402 is further configured to classify the parameter data according to the classification result, to obtain at least one group of candidate parameter data respectively corresponding to various types of ablation objects; and exclude parameter data in the candidate parameter data where the characteristic change data of the ablation site does not reach a preset change standard, to obtain the at least one group of target parameter data as the ablation parameter configuration scheme.

Further, the apparatus further includes:
a setting module 501, configured to set an average of the characteristic change data of the ablation sites as the preset change standard.

Further, the setting module 501 is further configured to analyze normal distribution characteristics of the characteristic change data of the ablation site, and set a normal distribution characteristic value obtained after analysis as the preset change standard.

Further, the apparatus further includes:
a monitoring module 502, configured to monitor physiological changes of each of the ablation objects by an intelligent physical examination apparatus, to obtain physiological change data of each of the ablation objects after each of the associated ablation tasks is implemented; and
a screening module 503, configured to screen the target parameter data in the database periodically according to the physiological change data.

Further, the screening module 503 is further configured to obtain a physiologically abnormal change rate periodically according to the physiological change data, wherein the physiological change data includes at least one of body temperature, blood pressure, blood glucose, heart rate, blood lipid, vital capacity, and blood oxygen saturation; and adjust the storage state of the target parameter data in the database according to the physiologically abnormal change rate and a preset rate.

Further, the apparatus further includes:
a receiving module 504, configured to receive the identification information of the ablation object, imaging time, and imaging data of the ablation object sent from a medical imaging apparatus; and
a recognition module 505, configured to perform image recognition on the imaging data, generate characteristic data of the ablation site of the ablation object according to the recognition result, correlate the identification information of the ablation object and the imaging time with the characteristic data of the ablation site, and store the correlation relationship by the output module 403 in an ablation site information database.

The acquisition module 401 is further configured to query the ablation site information database to obtain the characteristic data of the ablation site of each of the ablation objects and the corresponding imaging time; and obtain the characteristic change data for the ablation site of each of the ablation objects according to the imaging time.

Further, the acquisition module 401 is further configured to acquire a first target data when a parameter analysis instruction is received. The first target data includes characteristic data of an ablation object, characteristic data of an ablation site, description data of an ablation task to be implemented, and parameter data to be configured indicated by the parameter analysis instruction.

The apparatus further includes:
a first determination module 506, configured to determine, according to the first target data and a preset first target classification condition, a type of the ablation object indicated by the parameter analysis instruction;
a first query module 507, configured to query, in the database, at least one group of parameter data matching the determined type as reference data; and
a feasibility analysis module 508, configured to perform feasibility analysis on the parameter data to be configured according to the reference data and output the analysis result by the output module 403.

Further, the acquisition module 401 is further configured to acquire a second target data when a parameter query instruction is received. The second target data includes characteristic data of an ablation object, characteristic data of an ablation site, and description data of an ablation task to be implemented indicated by the parameter query instruction.

The apparatus further includes:
a second determination module 509, configured to determine, according to the second target data and a preset second target classification condition, a type of the ablation object indicated by the parameter query instruction; and
a second query module 510, configured to query, in the database, at least one group of parameter data matching the determined type, and output the query result.

Further, the characteristic data includes at least one of the gender, the disorder before the ablation task is implemented and the survival time after the ablation task is implemented, and the age of the ablation object.

The description data of the ablation task includes description data of the implementation time of the ablation task and the ablation stage corresponding to the ablation task.

The characteristic change data of the ablation site includes the change data of at least one of size, shape and area of the ablation site before and after the associated ablation task is performed.

The specific process for the above modules to implement their respective functions can be made reference to the relevant description in the embodiments shown in FIG. 2 and FIG. 3, and will not be repeated here again.

In the embodiment of the present invention, radio frequency ablation data of each of the ablation tasks performed in a preset period of time is acquired, and the acquired radio frequency ablation data is analyzed, to obtain respective ablation parameter configuration schemes corresponding to various types of ablation objects. As a result, automatic analysis of radio frequency ablation data based on big data and query and analysis of an ablation parameter configuration scheme based on the automatic analysis are realized, so as to improve the utilization of the radio frequency ablation data. Moreover, the analysis result is obtained based on massive data, and thus has a high reference value.

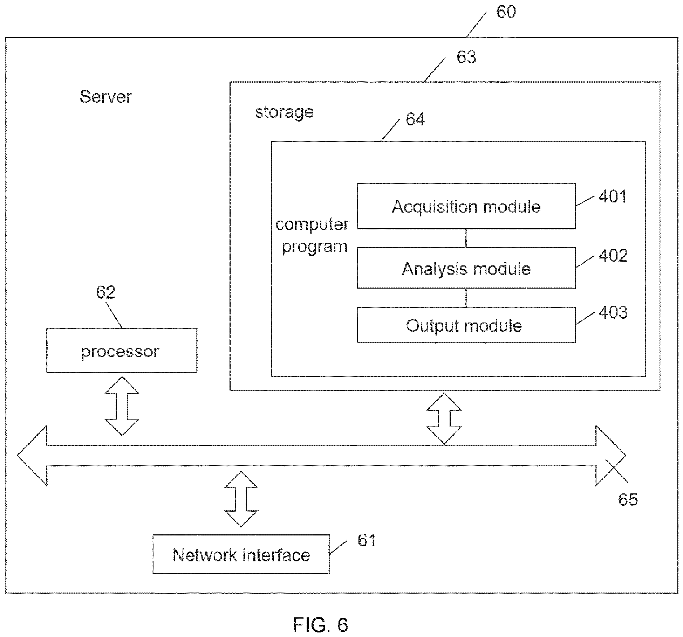

FIG. 6 is a schematic diagram showing a hardware structure in a server provided in an embodiment of the present invention. As shown in FIG. 6, a server 60 includes a network interface 61, a processor 62, a storage 63, a computer program 64 stored on the storage 63 and running on the processor 62, and a system bus 65. The system bus 65 is connected to the network interface 61, the processor 62 and the storage 63. When the computer program 64 is executed by the processor 62, steps in various embodiments of the radio frequency ablation data processing method, for example, Step S201 to Step S202 shown in FIG. 2, are implemented.

The network interface 61 is configured to communicate with other servers.

Exemplarily, the processor 62 may be a central processing unit (CPU) or other general-purpose processors, a digital signal processor (DSP), an application specific integrated circuit (ASIC), a field-programmable gate array (FPGA) or other programmable logic devices, a discrete gate or transistor logic device, a discrete hardware assembly, and others. A general-purpose processor can be a microprocessor or any conventional processors.

Exemplarily, the storage 63 is, for example, a hard drive storage, a non-transitory storage, a non-volatile storage (such as flash memory or other storages that are used to form solid-state drives and are electronically programmable to confine the deletion, etc.), and a volatile storage (such as static or dynamic random access storage), which is not limited in the embodiments of the present invention. The storage 63 can include both an internal storage unit of the server 60 and an external storage device. The storage 63 is configured to store the computer program and other programs and data required by the server 60. The storage 63 may also be configured to temporarily store data that have been outputted or will be outputted.

Exemplarily, the computer program 64 can be divided into one or more modules/units, stored in the storage 63 and implemented by the processor 62, to accomplish the present invention. The one or more modules/units can be a series of computer program instruction segments capable of implementing particular functions, and configured to describe an execution process of the computer program 64 by the server 60. For example, the computer program 64 is divided into an acquisition module 401, an analysis module 402 and an output module 403. The functions of various modules are as described below.

The acquisition module 401 is configured to acquire radio frequency ablation data of each of the ablation tasks performed in a preset period of time, wherein the radio frequency ablation data includes configured parameter data for each device in a radio frequency ablation system when each of the ablation tasks is performed, and characteristic data of an ablation object associated with each of the ablation tasks.

The analysis module 402 is configured to analyze the radio frequency ablation data, to obtain respective ablation parameter configuration schemes corresponding to various types of ablation objects.

The output module 403 is configured to output the ablation parameter configuration scheme.

The specific process for the above function modules to implement their functions can be made reference to relevant descriptions in embodiments shown in FIGs. 4 to 5, and will not be repeated here again.

Further, the storage 63 further stores a device driver program, which may be a network and interface driver program.

It can be understood by those skilled in the art that FIG. 6 is merely an example of the server 60, and does not constitute a limitation on the server 60. In practical use, more or fewer components, or a combination of some components, or different components can be included. For example, the server 60 may further include an input/output device (such as a keyboard, a microphone, a camera, a loudspeaker, and a display screen, etc.).

Further, an embodiment of the present invention further provides a non-transitory computer-readable storage medium. The non-transitory computer-readable storage medium can be configured in the server in each of the above embodiments, and a computer program is stored in the non-transitory computer-readable storage medium. When the program is executed by a processor, the radio frequency ablation data processing method according to the embodiments shown in FIG. 2 and FIG. 3 is implemented.

In the embodiments described above, emphasis has been placed on the description of various embodiments. Parts of an embodiment that are not described in detail may be found in the description of other embodiments.

Those of ordinary skill in the art will recognize that the exemplary modules/units and algorithm steps described in connection with the embodiments disclosed herein may be implemented by electronic hardware, or a combination of computer software and electronic hardware. Whether such functions are implemented by hardware or software depends on the particular application and design constraints of the technical solutions. Skilled artisans may implement the described functions in varying ways for each particular application. However, such implementations are not intended to exceed the scope of the present invention.

In the embodiments provided in the present invention, it can be understood that the disclosed device/terminal and method may be implemented in other ways. For example, the device/terminal embodiments described above are merely illustrative. For example, the division of modules or elements is only a division in logical functions, and there may be additional divisions in actual implementation. For example, multiple elements or components may be combined or integrated into another system, or some features may be omitted, or not performed. Alternatively, the couplings or direct couplings or communicative connections shown or discussed with respect to one another may be indirect couplings or communicative connections via some interfaces, devices or units, and may be electrical, mechanical or otherwise.

The units described as separate components may or may not be physically separate, and the components shown as units may or may not be physical units, i.e. may be located in one place, or may be distributed over a multiple network elements. Some or all of the units may be selected to achieve the objectives of the solution of the present embodiment according to practical requirements.

In addition, the functional units in the various embodiments of the present invention may be integrated into one processing unit, may be physically separate from each other or may be integrated in one unit by two or more units. The integrated units described above can be implemented either in the form of hardware, or software functional units.

The integrated unit, if implemented in the form of a software functional unit and sold or used as a stand-alone product, may be stored in a computer-readable storage medium. Based on such an understanding, all or part of the processes of the methods in the above-described embodiments implemented in the present invention may also be implemented by a computer program instructing related hardware. The computer program may be stored in a computer-readable storage medium, and the steps of the various method embodiments described above are implemented when the computer program is executed by a processor. The computer program includes a computer program code, which may be in the form of source code, object code, or executable file, or in some intermediate form. The computer readable medium may include: any entity or device capable of carrying the computer program code, recording media, U disks, movable hard disks, magnetic disks, optical disks, computer memory, read-only memory (ROM), random access memory (RAM), electrical carrier wave signals, and telecommunications signals and software distribution media etc. It should be noted that the computer readable medium may contain content that may be appropriately augmented or subtracted as required by legislation and patent practice within judicial jurisdictions. For example, the computer-readable medium does not include electrical carrier wave signals and telecommunications signals in accordance with legislation and patent practices in some jurisdictions

The above-described embodiments are merely illustrative of, and not intended to limit the technical solutions of the present invention. Although the present invention has been described in detail with reference to the foregoing embodiments, those skilled in the art will appreciate that the technical solutions of the above-mentioned embodiments can still be modified, or some of the technical features therein can be equivalently substituted. Such modifications and substitutions do not cause the nature of the corresponding technical solution to depart from the spirit and scope of the embodiments of the present invention, and are intended to be included within the scope of the present invention.

## Claims

1. A radio frequency ablation data processing method, for use in a computer device, comprising steps of:
acquiring radio frequency ablation data of each of the ablation tasks performed in a preset period of time, wherein the radio frequency ablation data comprises configured parameter data for each device in a radio frequency ablation system when each of the ablation tasks is performed, and characteristic data of an ablation object associated with each of the ablation tasks; and
analyzing the radio frequency ablation data, to obtain and output respective ablation parameter configuration schemes corresponding to various types of ablation objects.

2. The method according to claim 1, wherein the radio frequency ablation data further comprises description data of each of the ablation tasks and characteristic change data of the ablation site of each of the ablation objects; and
the step of analyzing the radio frequency ablation data to obtain and output respective ablation parameter configuration schemes corresponding to various types of ablation objects comprises:
classifying the ablation object according to the characteristic data of the ablation object, the description data of the ablation task, the characteristic change data of the ablation site, and a preset classification condition;
analyzing the parameter data according to the classification result and the characteristic change data of the ablation site, to obtain at least one group of target parameter data corresponding to each of the various types of ablation objects as the ablation parameter configuration scheme; and
establishing a correlation relationship of each of the various type of ablation objects with the respective corresponding classification condition and the at least one group of respective matching target parameter data, and outputting and storing the established correlation relationship in a database.

3. The method according to claim 2, wherein the step of analyzing the parameter data according to the classification result and the characteristic change data of the ablation site, to obtain at least one group of target parameter data corresponding to each of the various types of ablation objects as the ablation parameter configuration scheme, comprises:
classifying the parameter data according to the classification result, to obtain at least one group of candidate parameter data corresponding to each of the various types of ablation objects; and
excluding parameter data in the candidate parameter data where the characteristic change data of the ablation site does not reach a preset change standard, to obtain the at least one group of target parameter data as the ablation parameter configuration scheme.

4. The method according to claim 3, further comprising:
setting an average of the characteristic change data of the ablation sites as the preset change standard.

5. The method according to claim 3, further comprising:
analyzing normal distribution characteristics of the characteristic change data of the ablation site, and setting a normal distribution characteristic value obtained after analysis as the preset change standard.

6. The method according to claim 2, further comprising:
monitoring physiological changes of each of the ablation objects by an intelligent physical examination apparatus, to obtain physiological change data of each of the ablation objects after each of the associated ablation tasks is implemented; and
screening the target parameter data in the database periodically according to the physiological change data.

7. The method according to claim 6, wherein the step of screening the target parameter data in the database periodically according to the physiological change data comprises:
obtaining a physiologically abnormal change rate periodically according to the physiological change data, wherein the physiological change data comprises at least one of body temperature, blood pressure, blood glucose, heart rate, blood lipid, vital capacity, and blood oxygen saturation; and
adjusting the storage state of the target parameter data in the database according to the physiologically abnormal change rate and a preset rate.

8. The method according to claim 2, further comprising:
receiving the identification information of the ablation object, imaging time, and imaging data of the ablation object sent from a medical imaging apparatus;
performing image recognition on the imaging data, and generating characteristic data of the ablation site of the ablation object according to the recognition result; and
correlating the identification information of the ablation object and the imaging time with the characteristic data of the ablation site, and storing the correlation relationship in an ablation site information database; and
the step of acquiring characteristic change data of the ablation site of each of the ablation objects comprises:
querying the ablation site information database to obtain the characteristic data of the ablation site of each of the ablation objects and the corresponding imaging time; and
obtaining the characteristic change data for the ablation site of each of the ablation objects according to the imaging time.

9. The method according to claim 2, further comprising:
acquiring a first target data when a parameter analysis instruction is received, wherein the first target data comprises characteristic data of an ablation object, characteristic data of an ablation site, description data of an ablation task to be implemented, and parameter data to be configured indicated by the parameter analysis instruction;
determining, according to the first target data and a preset first target classification condition, the type of the ablation object indicated by the parameter analysis instruction;
querying, in the database, at least one group of parameter data matching the determined type as reference data; and
performing feasibility analysis on the parameter data to be configured according to the reference data and outputting the analysis result.

10. The method according to claim 2, further comprising:
acquiring a second target data when a parameter query instruction is received, wherein the second target data comprises characteristic data of an ablation object, characteristic data of an ablation site, and description data of an ablation task to be implemented indicated by the parameter query instruction;
determining, according to the second target data and a preset second target classification condition, the type of the ablation object indicated by the parameter query instruction;
querying, in the database, at least one group of parameter data matching the determined type and outputting the query result.

11. The method according to any one of claims 1 to 10, wherein the characteristic data comprises at least one of the gender, the disorder before the ablation task is implemented and the survival time after the ablation task is implemented, and the age of the ablation object;
the description data of the ablation task comprises description data of the implementation time of the ablation task and the ablation stage corresponding to the ablation task; and
the characteristic change data of the ablation site comprises the change data of at least one of the size, shape and area of the ablation site before and after the associated ablation task is performed.

12. A radio frequency ablation data processing apparatus, comprising:
an acquisition module, configured to acquire radio frequency ablation data of each of the ablation tasks performed in a preset period of time, wherein the radio frequency ablation data comprises configured parameter data for each device in a radio frequency ablation system when each of the ablation tasks is performed, and characteristic data of an ablation object associated with each of the ablation tasks;
an analysis module, configured to analyze the radio frequency ablation data, to obtain respective ablation parameter configuration schemes corresponding to various types of ablation objects; and
an output module, configured to output the ablation parameter configuration scheme.

13. A server, comprising:
a storage and a processor, wherein
the storage stores an executable program code; and
the processor is coupled to the storage, and configured to call the executable program code stored in the storage, and implement the radio frequency ablation data processing method according to any one of claims 1 to 11.

14. A non-transitory computer-readable storage medium, storing a computer program, wherein when the computer program is executed by a processor, the radio frequency ablation data processing method according to any one of claims 1 to 11 is implemented.
